# EUROPÄISCHE PATENTSCHRIFT

(11) **EP 0 536 610 B1**
(45) Veröffentlichungstag und Bekanntmachung des Hinweises auf die Patenterteilung: **03.09.1997**
(21) Anmeldenummer: 92116501.5
(22) Anmeldetag: 26.09.1992
(51) Int. Cl.: A61F 2/06

(54) **Vorrichtung zum Aufweiten einer Stenose**
Stenosis dilatation device
Dispositif pour la dilatation d'une sténose

(30) Priorität: 10.03.1992 DE 4207557; 11.10.1991 DE 4133696
(43) Veröffentlichungstag der Anmeldung: 14.04.1993
(73) Patentinhaber: ANGIOMED GmbH & Co. Medizintechnik KG, 76227 Karlsruhe (DE)
(72) Erfinder: Lindenberg, Josef, W-7500 Karlsruhe 41 (DE); Schnepp-Pesch, Wolfram, W-7500 Karlsruhe 41 (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 380 668
- EP-A- 0 411 118
- FR-A- 2 617 721
- US-A- 4 665 918
- US-A- 4 681 110

## Beschreibung

Die Erfindung betrifft eine Vorrichtung zum Aufweiten einer Stenose nach dem Oberbegriff des Anspruchs 1. Eine solche Vorrichtung ist z.B. aus der EP-A-380668 bekannt.

Ein Verschluß in einem Gefäß, wie in einer Arterie, wird zunächst in der Regel erweitert, indem Ablagerungsmaterial, wie Plaques, entfernt werden, beispielsweise durch Herausschneiden und Absaugen oder dergleichen. Dies kann aber nicht bis zum Gewebematerial des Gefäßes selbst geschehen, da hier eine Verletzungsgefahr besteht.

Wenn lediglich eine Verengung, eine Stenose, aber kein völliger Verschluß vorliegt, wie dies beispielsweise auch bei vergrößerter Prostata im Bereich der Harnröhre der Fall ist, oder nachdem ein gewisser Durchflußbereich bei vorherigem vollständigem Verschluß in der vorgenannten Weise geschaffen wurde, ist eine radiale Aufweitung der Stenose erwünscht. Eine solche wird daher nicht nur in Blutgefäßen, sondern auch in anderen Körpergängen oder -röhren, wie im Harnleiter, in der Harnröhre, in Gallengängen oder dergleichen, vorgenommen.

Hierzu wurde bisher ein sogenannter Ballonkatheter, gegebenenfalls mit einem doppelten Lumen verwendet, der eine momentane radiale Aufweitung bewirkt. Diese Aufweitung der Stenose als solche sollte dauerhaft bestehen bleiben, wenn der Ballonkatheter wieder entfernt wurde. Dies ist in der Regel aber nicht der Fall. Es wurden daher auch schon permanente Aufweitungen einer Stenose vorgeschlagen. So soll auf einem Ballonkatheter ein Aufweiteteil axial fest aufsitzen, mit dem Katheter eingeführt und durch Aufweiten des Ballonkatheters plastisch derart verformt werden, daß die radialen Abmessungen des Aufweiteteils sich vergrößern, dieses in die Wand der Stenose eingedrückt wird und nach Ablassen der Luft aus dem Ballonkatheter dieser wieder entfernt werden kann, während das Aufweiteteil im plastisch verformten Zustand an seinem Ort liegen bleibt. Als solches wurde beispielsweise eine Manschette in Form einer (gegebenenfalls durchbrochenen) Hülse aus Kunststoff oder Gewebe kompatiblem herkömmlichem Metall vorgesehen.

Ein wesentlicher Nachteil des letztgenannten Verfahrens besteht darin, daß die Manschette auf dem Außenumfang eines Katheters aufsitzend in die Stenose eingebracht werden muß. Zunächst ist die axiale Festlegung aufwendig und entweder nicht zuverlässig zu erreichen, so daß das Teil beim Vorschieben des Katheters zurückbleiben kann oder aber ein späteres Lösen des Teils vom Katheter ist schwierig. Darüber hinaus kann ein außen auf einem Katheter sitzendes Aufweiteteil auch zu Beschädigungen führen. Schließlich muß die Aufweitung durch den Ballonkatheter bei einem derartigen Teil zunächst zu einer Radialerstreckung hin erfolgen, die wesentlich über der letztendlich wünschenswerten und zu erreichenden Radialerstreckung des Aufweiteteils liegt, da solche Teile, wie herkömmliche kompatiblen Materialien, soweit sie eine ausreichende Festigkeit haben, um eine Stenose offen zu halten, einen erheblichen elastischen Verformungsbereich aufweisen, bevor eine bleibende plastische Verformung auf den gewünschten Radius eintritt. Dies gilt insbesondere, wenn die Stenose bzw. das umgebende Gewebematerial von außen radial gegen das Aufweiteteil drückt.

Die EP 411 118 A1 und die EP 380 668 A1 zeigen eine gattungsgemäße Vorrichtung, bei der eine schraubenförmig gewundene Endoprothese in ihrer Tieftemperaturstellung auf einem Applikator mit Einbuchtungen aufsitzt. Die Endoprothese wird - aufsitzend auf dem Applikator - durch einen Führungskatheter bis in den Bereich der zu behebenden Stenose eingebracht. Damit die Endoprothese sich nicht vorzeitig in ihre Hochtemperaturstellung mit radial größerem Durchmesser aufweitet, wobei dann der Applikator nicht mehr zuverlässig an ihr angreifen könnte, muß sie permanent gekühlt werden. Hierzu ist der Applikator als Katheterrohr ausgebildet, das im Bereich der Endoprothese in der Wandung eine Vielzahl von Öffnungen aufweist, durch welche eine Kühlflüssigkeit herausgedrückt wird. Es ist nachteilig, wenn in Körpergefäße solch eine Kühlflüssigkeit als Fremdkörper eingeführt wird. Die FR 2 617 721 zeigt eine Endoprothese auf einem verjüngten Zwischenteil eines Ballonkatheters, dessen Ballonteil sich vor der Endoprothese befindet. Durch den Ballon erfolgt ein Absperren des Blutstromes im Gefäß. Durch einen umgebenden Einführkatheter wird zum Aufweiten der Endoprothese eine Flüssigkeit mit einer Temperatur von bis zu 50° eingeführt, um in dieser Weise die Endoprothese, die eine relativ hohe Übergangstemperatur oberhalb der normalen Körpertemperatur hat, aufzuweiten.

Der Erfindung liegt die Aufgabe zugrunde, eine Vorrichtung zum Aufweiten einer Stenose zu schaffen, bei der unter Vermeidung der vorgenannten Nachteile ein einfaches, unproblematisches und zuverlässiges Aufweiten einer Stenose in kurzer Zeit möglich ist.

Erfindungsgemäß wird die genannte Aufgabe mit einer Vorrichtung zum Aufweiten einer Stenose der eingangs genannten Art gelöst, welche das Kennzeichen des Anspruchs 1 aufweist.

Die Endoprothese aus Formgedächtnis-Legierung kann dabei in unterschiedlichster Weise ausgebildet sein. Es kann ein in seiner Tieftemperaturstellung spiralförmig gewickeltes Blatt sein, das sich in der Hochtemperaturstellung zu einem Zylindermantel aufweitet. Es kann ein Streckmetall sein, das Rhomben- oder bienenwabenförmige Durchbrechungen aufweist. Es kann ein Teil aus schraubgewendeltem Draht sein, wobei gegebenenfalls die Einzelwindungen mit achsparallelen Ausbiegungen versehen sind, die jeweils mit einer benachbarten Bindung (durch Lötung) fest verbunden sind, wobei die Festigkeit der Verbindungen unter der Reißfestigkeit des die Endoprothese bildenden Faden liegt, vorzugsweise aber über den aufzuwendelnden Biegekräften, insbesondere um die Wendel zu längen. Darüber hinaus können die Endoprothesen aus Geweben, wie Kreuzgeweben, oder Gestricken, wie insbesondere Milanese-Rund-Gestricken bestehen. Schließlich kann eine Endoprothese mit einem achsparallelen Verbindungsteil (Rückgrat) gebildet sein, von dem sich bogenförmige Rippen beidseitig erstrecken, die jeweils von einer zur anderen Seite hin in axialer Richtung versetzt sind, so daß ihre freien Enden zwischeneinander greifen.

Gemäß bevorzugter Ausgestaltung ist vorgesehen, daß Applikator und Endoprothese sich zumindestens teilweise axial überlappend angeordnet sind.

In allen Fällen liegt das Innenlumen der Schleuse insbesondere nicht unter dem Außenlumen der Endoprothese in deren Tieftemperaturzustand, also in deren Zustand unterhalb der Umgebungstemperatur, ab der sich die Endoprothese in ihren Hochtemperaturzustand aufweitet. Der Gegenhalter oder Schieber weist gegebenenfalls einen Durchmesser auf, der dem der Endoprothese in ihrem Niedrigtemperaturzustand im wesentlichen entspricht.

Wesentlich ist bei der Erfindung weiterhin, daß die Endoprothese vor dem Einbringen an den Applikationsort zuverlässig und fest radial von außen in ihrer komprimierten Tieftemperaturstellung gehalten wird, was durch die sie umgebende Schleuse gewährleistet ist. Um vor dem Einbringen der Endoprothese an ihren Applikationsort ein unbeabsichtigtes auch nur teilweises Herausschieben der Endoprothese aus der Schleuse zu vermeiden, was den weiteren Einsatz unmöglich machen würde, ist eine bevorzugte Ausgestaltung dadurch gekennzeichnet, daß Schleuse und Applikator bzw. Gegenhalter lösbar axial blockiert sind, wobei hierzu ein Schleuse und Applikator axial blockierendes, lösbares Blockierelement vorgesehen ist. In vorteilhafter Weiterbildung kann vorgesehen sein, daß Schleuse und Applikator mit axial relativ zueinander beweglichen Griffteilen versehen sind. Eine äußerst einfache Ausgestaltung sieht dabei vor, daß das Blockierelement zwischen den beiden Griffteilen angeordnet ist, wobei weiterhin das Blockierelement ein zwischen den Griffteilen entnehmbar angeordneter Abstandhalter ist.

Um die Endoprothese über den Applikator, der durch sein Griffteil gehalten wird, zuverlässig am Applikationsort zu halten und bei dem Entfernen der Schleuse nicht auch eine Relativbewegung des Applikators zu bewirken, ist vorgesehen, daß die Schleuse relativ zum mit dem Applikator verbundenen Griffteil axial verschiebbar ist, wobei in äußerst bevorzugter Ausgestaltung zusätzlich eine Axialführung für das mit der Schleuse verbundene Griffteil relativ zum mit dem Applikator verbundenen Griffteil vorgesehen sein kann.

Weitere Ausgestaltungen sehen vor, daß der Applikator und mit ihm fest verbundene Teile hohl ausgebildet sind. Hierdurch kann ein Führungsdraht durch die gesamte Vorrichtung hindurchgeschoben werden bzw. die Vorrichtung nach Legen eines Führungsdrahts bis in das zu behandelnde Gefäß und zu dessen Stenose über einem Führungsdraht eingeführt werden kann. In weiterer Ausbildung ist vorgesehen, daß am rückwärtigen Ende des Applikators bzw. ein mit diesem fest verbundenen Teil (Griffteil) eine Adaptereinrichtung vorgesehen ist. Hierdurch kann eine Spritze angebracht werden, mit der beispielsweise plaquelösende Mittel (Streptokinase) eingespritzt werden können, wenn dies erforderlich sein sollte.

Eine Weiterbildung sieht vor, daß am rückwärtigen Ende der Schleuse ein Sauganschluß ausgebildet ist.

Weitere Vorteile und Merkmale der Erfindung ergeben sich aus den Ansprüchen und aus der nachfolgenden Beschreibung, in der ein Ausführungsbeispiel der erfindungsgemäßen Vorrichtung unter Bezugnahme auf die Zeichnung im einzelnen erläutert ist. Dabei zeigt:

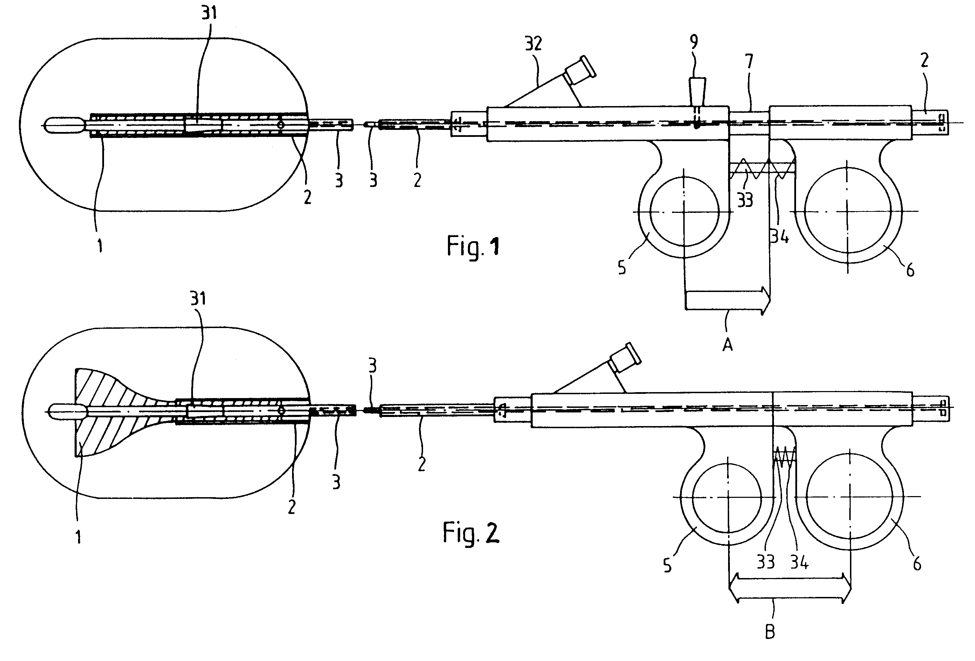

- Figur 1: eine bevorzugte Ausgestaltung der erfindungsgemäßen Vorrichtung in schematischer Darstellung, wobei das vordere Ende vergrößert dargestellt ist; und
- Figur 2: die Ausgestaltung der Fig. 1 mit teilweise aus der Schleuse herausgeschobener Endoprothese.

Die erfindungsgemäße Vorrichtung zum Aufweiten einer Stenose in einer Körperröhre weist zunächst eine Endoprothese 1 aus einer Formgedächtnis-Legierung (mit Memory-Effekt), wie Ti-Ni-, Al-Ni-, Cu-Zn-Legierung auf, wie sie unter eingetragenen Handelsnamen Nitinol, Biometall oder Memotall bekannt sind. Die Endoprothese 1 wird mit einem geringen radialen Durchmesser durch eine Hülse 2 gehalten und weist in dieser Stellung im wesentlichen zylindrische Außenkontur auf. Die Endoprothese 1 ist in der Zeichnung schematisch dargestellt.

Bei der dargestellten Ausführungsform weist die erfindungsgemäße Vorrichtung zum Legen einer Endoprothese 1 eine Schleuse 2 auf, die mit einem ersten Griffteil 5 verbunden bzw. verbindbar ist. In eine durch eine Axialbohrung gebildete Führung des Griffteils 5 erstreckt sich ein Ansatz 7 eines weiteren Griffteils 6, welches einen Applikator 3 trägt, der durch die Bohrung des Griffteils 5 und die Schleuse 2 hindurchragt und aus deren vorderen Ende heraustritt. Im vorderen Bereich der Schleuse 2 ist die Endoprothese 1 in Form eines zylindrischen Memory-Metallteils angeordnet, wobei sich der Applikator 3 ebenfalls durch die Schleuse 2 hindurcherstreckt. Der Applikator 3 ist in einem Bereich innerhalb der Schleuse 1, aber etwa in dessen rückwärtiger Hälfte mit einem radial elastischen Schieberabschnitt 31 versehen, der vorzugsweise durch Laschen ausgebildet sein kann, wobei der Schieberabschnitt 31 sich vom vorderen Ende zum rückwärtigen Ende hin verjüngt, d.h. gegebenenfalls die entsprechenden Laschen am rückwärtigen Ende fest mit dem Applikator 3 verbunden sind, während ihre vorderen Enden frei sind und sich radial nach außen aufstellen können, so daß sie an der Endoprothese 1 anliegen bzw. in deren Maschen eingreifen.

Am Griffteil 5 ist ein Sauganschluß 32 vorgesehen, welcher mit dem Inneren der Schleuse 2 in Verbindung steht, so daß an den Sauganschluß 32 eine Saugeinrichtung, wie eine Spritze oder eine Pumpe, angeschlossen werden kann mittels der über die Schleuse 2 aus deren vorderen Ende Flüssigkeit und gegebenenfalls Thrombenteile oder dergleichen abgesaugt werden können.

Weiterhin ist das Griffteil 5 mit einem Blockierelement 9 in Form eines Stiftes versehen, der radial in das Griffteil 5 und in den Ansatz 7 des Griffteils 6 einsteckbar ist, um beide Griffteile 5 und 6 und damit ebenfalls die Schleuse 2 und der Applikator 3 in axialer Richtung zu blockieren. Dies ist der Lieferzustand der erfindungsgemäßen Vorrichtung. Hierdurch wird ein unbeabsichtigtes Herausschieben der Endoprothese aus der Schleuse 2 vor dem Einbringen des vorderen Endes der Vorrichtung in den aufzuweitenden Bereich zuverlässig verhindert.

Die Griffteile sind im dargestellten Ausführungsbeispiel in Form von zwei Griffringen ausgebildet, in die mit Daumen und Finger eingegriffen werden kann, so daß durch eine abzugsähnliche Hin- und Herbewegung, wie sie durch die Pfeile A und B angedeutet ist, die Griffteile 5, 6 und damit die Schleuse 2 und der Applikator 3 axial relativ zueinander hin- und herbewegt werden können, nachdem das Blockierelement 9 entfernt wurde. Damit die Griffteile automatisch auseinander gedrückt werden und daher vom Benutzer nur gegeneinander gedrückt werden müssen, ist zwischen ihnen auf einem Führungsbolzen 33 eine Schraubenfeder 34 angeordnet, die bewirkt, daß die Endoprothese 1 automatisch in den Applikator 3 zurückbewegt wird, wenn der Benutzer keine Kraft ausübt.

Die erfindungsgemäße Vorrichtung bzw. die Schleuse 2 mit der in ihr befindlichen Endoprothese 1 und dem sich durch sie hindurcherstreckenden Applikator 3 werden in an sich üblicher Weise in das aufzuweitende Gefäß eingebracht, beispielsweise durch einen in Seldinger-Technik gelegten Katheter. Wenn das vordere Ende der Schleuse 3 sich im Bereich der aufzuweitenden Stenose befindet bzw. insbesondere der Bereich der Schleuse 2, über den sich in ihr die Endoprothese erstreckt, innerhalb der Stenose liegt, kann nach Entfernen des Blockierelements 9 das die Schleuse 2 haltende Griffteil 5 gegen das Griffteil 6 zurückgezogen werden. Hierdurch wird über das Griffteil 6, den Applikator 3 und dessen Schieberansatz 31 die Endoprothese 1 relativ zur Schleuse 2 an ihrem axialen Ort gehalten, während die Schleuse 2 mit ihrem vorderen Ende von der Endoprothese 1 teilweise zurückgeschoben wird und damit das vordere Ende der Endoprothese 1 freigibt, welches sich innerhalb der Stenose aufweiten kann, wie dies in Figur 8 dargestellt ist. Anschließend wird das Griffteil 6 ein Stück zurückgezogen und damit vom Griffteil 5 gelöst. Hierdurch wird der Applikator 3 mit dem Schiebeelement 31 ebenfalls innerhalb der Endoprothese 1 und der Schleuse 2 zurückgezogen. Aufgrund der Ausgestaltung des Schiebeabschnitts 31 mit der beschriebenen Teilkonusform gleitet dieses innerhalb der Endoprothese 1 an dieser nach rückwärts entlang, während die Endoprothese 1 selbst aufgrund der höheren Reibkraft innerhalb der Schleuse 2 axial festgehalten wird. In einem weiteren Schritt wird mittels des Griffteils 5 die Schleuse 2 wieder zurückgezogen. Die freien Enden des Schiebeteils 31 liegen dabei, wie gesagt, an der Endoprothese 1 an bzw. greifen teilweise in deren Maschen oder Zwischenräume ein, so daß sie wiederum ein Mitzurückziehen mit der Schleuse 2 verhindern, vielmehr die Endoprothese 1 an ihrem axialen Ort halten.

Dieser Vorgang wird so lange wiederholt, bis die gesamte Endoprothese aus der Schleuse 2 freigegeben ist und frei im Stenosenbereich liegt und diesen aufweitet. Die Aufweitung der Endoprothese 1 geschieht aufgrund des Umstandes, daß diese, wie gesagt, aus Memory-Metall besteht, dessen Hochtemperaturstellung die aufgeweitete Stellung ist, während die Niedertemperaturstellung etwa der Ausgestaltung entspricht, bei der sie in der Figur 7 sich vollständig innerhalb der Schleuse 2 befindet.

Diese Ausgestaltung einer erfindungsgemäßen Vorrichtung ist äußerst einfach und einfach in der Anwendung.

Die Endoprothese selbst kann vielfältige Form aufweisen, wie schraubgewendelter Draht oder spiralig gerolltes Blech, das sich beim Aufweiten aus der Tief- in die Hochtemperaturphase zu einem aufgeweiteten Zylindermantel bildet. Sie kann aus zylindermantelförmigem Streckmetall, einem Rundgewebe (Kreuzgewebe) oder Gestricke (Milanese-Gestricke) bestehen. Sie kann gerippeartig mit einem achsparallelen Rückgrat oder Verbindungssteg ausgebildet sein, von dem sich teilringförmig gebogene Rippen erstrecken. Diese können zu dem Verbindungssteg im rechten Winkel oder aber in einem vom rechten Winkel abweichenden Winkel, wie 50 bis 70°, ausgerichtet sein. Vorzugsweise sind jeweils entgegengesetzt von dem Verbindungssteg sich erstreckende Rippen axial zueinander versetzt angeordnet, so daß die freien Enden der Rippen einer Seite jeweils zwischen die freien Enden der sich zur anderen Seite hin erstreckenden Rippen eingreifen können.

Wenn die Endoprothese wendelförmig ausgebildet ist, so kann sie mit achsparallel ausgerichteten Ausbiegungen versehen sein, von denen ein Teil oder die sämtlich mit jeweils einer benachbarten Wendelwindung fest verbunden sind, wie durch Löten. In diesem Falle weist die Lötverbindung eine geringere Lösekraft auf, als sie der Reißkraft des die Wendel bildenden Drahtes entspricht. Wenn an einem freien Ende des die Wendel bildenden Drahtes eine Kugel angebracht ist, so kann an dieser mittels einer Hohlzange angegriffen und die gesamte Endoprothese auch wieder entfernt werden, wenn dies notwendig werden sollte. Soweit nicht sämtliche Wendeln in der beschriebenen Weise miteinander verbunden sind, kann die Länge der Wendel durch Stauchen oder Auseinanderziehen an den zu behandelnden Stenosenbereich angepaßt werden.

## Patentansprüche

1. Vorrichtung zum Aufweiten einer Stenose in einer Körperröhre, wie in einer Arterie, im Harnleiter, in der Harnröhre, in einem Gallengang oder dergleichen, mit einer Endoprothese (1) aus einer Formgedächtnislegierung mit im wesentlichen zylindermantelförmiger Außenkontur, mit einer die Endoprothese umgebenden Schleuse (2) und mit einem innerhalb der Schleuse axial verschiebbaren, sich bis in die Endoprothese erstreckenden Applikator (3) für die Endoprothese, welcher durch die Schleuse (2) radial zusammengehalten wird, dadurch gekennzeichnet, daß der Applikator (3) an seinem in die Endoprothese (1) ragenden Bereich einen Schieberabschnitt (31) aufweist, der sich von seinem vorderen zum hinteren Ende hin verjüngt.

2. Vorrichtung nach Anspruch 1, dadurch gekennzeichnet, daß Applikator (3) und Endoprothese sich zumindestens teilweise axial überlappend angeordnet sind.

3. Vorrichtung nach Anspruch 1 oder 2, dadurch gekennzeichnet, daß der Applikator sich in die Endoprothese hineinerstreckt.

4. Vorrichtung nach einem der vorangehenden Ansprüche, dadurch gekennzeichnet, daß die Schleuse (2) relativ zum Applikator (3) axial bewegbar ist.

5. Vorrichtung nach Anspruch 4, dadurch gekennzeichnet, daß Schleuse (2) und Applikator (3) lösbar gegen Relativbewegung axial blockiert sind.

6. Vorrichtung nach Anspruch 5, gekennzeichnet durch ein Schleuse (2) und Applikator (3) axial blockierendes, lösbares Blockierelement.

7. Vorrichtung nach einem der vorangehenden Ansprüche, dadurch gekennzeichnet, daß Schleuse (2) und Applikator (3) mit axial relativ zueinander beweglichen Griffteilen (5, 6) versehen sind.

8. Vorrichtung nach Anspruch 7, gekennzeichnet durch eine Axialführung für das mit der Schleuse (2) verbundene Griffteil (5) relativ zum mit dem Applikator (3) verbundenen Griffteil (6).

9. Vorrichtung nach einem der Ansprüche 6 bis 8, dadurch gekennzeichnet, daß das Blockierelement (9) zwischen den beiden Griffteilen (5, 6) angeordnet ist.

10. Vorrichtung nach Anspruch 9, dadurch gekennzeichnet, daß das Blockierelement (9) ein zwischen den Griffteilen (5, 6) entnehmbar angeordneter Abstandhalter ist.

11. Vorrichtung nach einem der vorangehenden Ansprüche, dadurch gekennzeichnet, daß der Applikator (3) über seine Länge hohl ausgebildet ist.

12. Vorrichtung nach einem der vorangehenden Ansprüche, dadurch gekennzeichnet, daß am rückwärtigen Ende des Applikators (3) bzw. an einem mit diesem fest verbundenen Teil eine Adaptereinrichtung vorgesehen ist.

13. Vorrichtung nach einem der vorangehenden Ansprüche, dadurch gekennzeichnet, daß am rückwärtigen Ende der Schleuse (2) ein Sauganschluß (32) ausgebildet ist.

14. Vorrichtung nach einem der vorangehenden Ansprüche, dadurch gekennzeichnet, daß zwischen Endoprothese (1) und Schleuse (2) eine Feder (34) angeordnet ist.

## Claims

1. Device for dilating a stenosis in a body tube, such as in an artery, in the ureter, in the urethra, in a bile duct or the like, with an endoprosthesis (1) of shape memory alloy with a substantially cylinder jacket-shaped outer contour, with a lock (2) surrounding the endoprosthesis and with an applicator (3) for the endoprosthesis extending in axially displaceable manner within the lock into the endoprosthesis, which is radially held together by the lock (2), characterized in that in its region projecting into the endoprosthesis (1), the applicator (31) has a slider section (31), which tapers from the front to the rear end.

2. Device according to claim 1, characterized in that the applicator (3) and endoprosthesis are arranged in an at least partly axially overlapping manner.

3. Device according to claim 1 or 2, characterized in that the applicator extends into the endoprosthesis.

4. Device according to one of the preceding claims, characterized in that the lock (2) is axially movable relative to the applicator (3).

5. Device according to claim 4, characterized in that the lock (2) and applicator (3) are axially detachably blocked against relative movement.

6. Device according to claim 5, characterized by a detachable blocking element axially blocking the lock (2) and applicator (3).

7. Device according to one of the preceding claims, characterized in that the lock (2) and applicator (3) are provided with handle parts (5, 6) movable axially relative to one another.

8. Device according to claim 7, characterized by an axial guide for the handle part (5) connected to the lock (2) relative to the handle part (6) connected to the applicator (3).

9. Device according to one of the claims 6 to 8, characterized in that the blocking element (9) is located between the two handle parts (5, 6).

10. Device according to claim 9, characterized in that the blocking element (9) is a spacer removably located between the handle parts (5, 6).

11. Device according to one of the preceding claims, characterized in that the applicator (3) is hollow over its entire length.

12. Device according to one of the preceding claims, characterized in that on the rear end of the applicator (3) or on a part firmly connected thereto is provided an adaptor means.

13. Device according to one of the preceding claims, characterized in that a suction connection (32) is formed on the rear end of the lock (2).

14. Device according to one of the preceding claims, characterized in that a spring (34) is positioned between the endoprosthesis (1) and the lock (2).

## Revendications

1. Dispositif pour dilater une sténose dans un conduit corporel, par exemple dans une artère, dans l'uretère, l'urètre, le canal cholédoque ou similaires, comprenant une endoprothèse (1) en alliage à mémoire de forme à profil extérieur sensiblement en forme de gaine cylindrique, un sas (2) entourant l'endoprothèse et un applicateur (3) pour l'endoprothèse déplaçable axialement à l'intérieur du sas, qui s'étend jusque dans l'endoprothèse et est maintenu radialement par le sas (2), caractérisé en ce que l'applicateur (3) présente dans sa zone pénétrant dans l'endoprothèse (1) un segment coulissant (31) qui se rétrécit de son extrémité avant vers son extrémité arrière.

2. Dispositif selon la revendication 1, caractérisé en ce que l'applicateur (3) et l'endoprothèse sont disposés de manière à se recouvrir axialement au moins partiellement.

3. Dispositif selon l'une quelconque des revendications 1 ou 2, caractérisé en ce que l'applicateur s'étend à l'intérieur de l'endoprothèse.

4. Dispositif selon l'une quelconque des revendications précédentes, caractérisé en ce que le sas (2) est déplaçable axialement par rapport à l'applicateur (3).

5. Dispositif selon la revendication 4, caractérisé en ce que le sas (2) et l'applicateur (3) sont bloqués contre leur déplacement relatif libérable.

6. Dispositif selon la revendication 5, caractérisé par un élément amovible de blocage axial du sas (2) et de l'applicateur (3).

7. Dispositif selon l'une quelconque des revendications précédentes, caractérisé en ce que le sas (2) et l'applicateur (3) sont munis de pièces de préhension (5,6) mobiles axialement l'une par rapport à l'autre.

8. Dispositif selon la revendication 7, caractérisé par un guidage axial de la pièce de préhension (5) reliée au sas (2) par rapport à la pièce de préhension (6) reliée à l'applicateur (3).

9. Dispositif selon l'une quelconque des revendications 6 à 8, caractérisé en ce que l'élément de blocage (9) est écarteur disposé amovible entre les pièces de préhension (5,6).

10. Dispositif selon la revendication 9, caractérisé en ce que l'élément de blocage (9) est un écarteur disposé amovible entre les pièces de préhension (5,6).

11. Dispositif selon l'une quelconque des revendications précédentes, caractérisé en ce que l'applicateur (3) est creux sur toute sa longueur.

12. Dispositif selon l'une quelconque des revendications précédentes, caractérisé en ce qu'on prévoit un dispositif d'adaptation à l'extrémité arrière de l'applicateur (3) ou à une pièce reliée fixe à celui-ci.

13. Dispositif selon l'une quelconque des revendications précédentes, caractérisé en ce qu'un raccord d'aspiration (32) est conformé à l'extrémité arrière du sas (2).

14. Dispositif selon l'une quelconque des revendications précédentes, caractérisé en ce qu'un ressort (34) est disposé entre l'endoprothèse (1) et le sas (2).
